(19) Europäisches Patentamt
European Patent Office
Office européen des brevets

(11) Veröffentlichungsnummer: **0 010 165**
**B1**

(12) # EUROPÄISCHE PATENTSCHRIFT

(45) Veröffentlichungstag der Patentschrift: **20.01.82**

(51) Int. Cl.³: **C 07 D 213/85**

(21) Anmeldenummer: **79103479.6**

(22) Anmeldetag: **17.09.79**

(54) **Verfahren zur Herstellung von 6-Hydroxypyrid-2-onen.**

(30) Priorität: **21.10.78 DE 2845863**

(43) Veröffentlichungstag der Anmeldung: **30.04.80 Patentblatt 80/9**

(45) Bekanntmachung des Hinweises auf die Patenterteilung: **20.01.82 Patentblatt 82/3**

(84) Benannte Vertragsstaaten: **CH DE FR GB IT NL**

(56) Entgegenhaltungen:
**DE-A-1 956 142**
**DE-A-2 307 445**
**FR-A-2 330 681**

**BERICHTE DER DEUTSCHEN CHEMISCHEN GESELLSCHAFT, Volume 26, Band IV, 1897, Seiten 654–656 . Berlin, DE. J. GUARESCHI et al.: «Synthesen von Pyridinverbindungen aus Ketonsäureäthern mittels Cyanessigäther bei Gegenwart von Ammoniak oder Aminen»**

(73) Patentinhaber: **CASSELLA Aktiengesellschaft, Hanauer Landstrasse 526, D-6000 Frankfurt am Main 61 (DE)**

(72) Erfinder: **Schmidt, Frank, Dr., Obermarkersdorfer Strasse 19, D-6454 Bruchköbel 1 (DE)**

(74) Vertreter: **Urbach, Hans-Georg, Dr. et al, Hanauer Landstrasse 526, D-6000 Frankfurt am Main 61 (DE)**

**EP 0 010 165 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 6-Hydroxypyrid-2-onen

Die Erfindung betrifft ein Verfahren zur Herstellung von 6-Hydroxypyrid-2-onen der allgemeinen Formel I

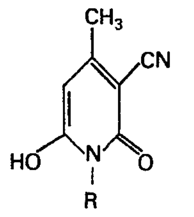

(I)

worin R für Wasserstoff oder einen gegebenenfalls verzweigten Alkylrest mit 1 bis 4 Kohlenstoffatomen steht, durch Umsetzung von Cyanacetamiden mit Acetessigsäureestern, bei dem man Cyanacetamide der allgemeinen Formel II

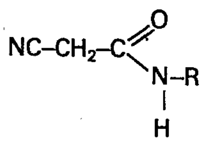

(II)

worin R die vorgenannte Bedeutung hat, oder ein im wesentlichen Cyanacetamide der allgemeinen Formel II enthaltendes Substanzgemisch mit Acetessigsäureestern der allgemeinen Formel III

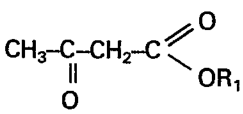

(III)

worin $R_1$ einen aliphatischen Rest mit 1 bis 8 Kohlenstoffatomen oder einen cycloaliphatischen Rest mit 3 bis 6 Kohlenstoffatomen bezeichnet, in Gegenwart von mindestens einem Mol eines Amins der allgemeinen Formel IV

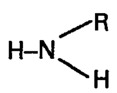

(IV)

worin R die vorgenannte Bedeutung hat, in wässriger Lösung oder Suspension unter einem Druck von 0,5 bis 50 bar auf Temperaturen zwischen 50 und 200 °C erwärmt.

Am Pyridonstickstoff unsubstituierte 6-Hydroxypyrid-2-one (allgemeine Formel I, R = Wasserstoff) sind seit langem bekannt. Sie sind zuerst von Guareschi bei der Umsetzung von β-Ketocarbonsäureestern mit Cyanacetamid gefunden und beschrieben worden (vgl. Mem. Accad. Torino cl. sci. fis. mat. e nat. [2], Band 46, Seite 11; Chemisches Zentralblatt 1896 I, 601). Andere Autoren haben diese Verbindung durch Cyclisierung des Umsetzungsprodukts von Acetessigsäureäthylester mit Malodinitril erhalten [z.B. Kasturi, T.R.; Sharma, V.K.; Srinivasan, A; Subrahmanyam, G.; Tetrahedron 29 (1973) 24, 4103-09 und Gudriniece, E.; Rigerte, B.; Latv. PSR Zinat. Akad. Vestis, Kim. Ser. (1974) (2), 239-40]. In der DE-OS 25 31 035 wird ein Verfahren zur Herstellung von 5-Cyanpyridonen-6 durch Umsetzung von Halogenessigsäureestern mit Stickstoffverbindungen und darauffolgenden Reaktionen des Umsetzungsgemisches mit Alkalicyaniden und dann mit Dicarbonylverbindungen beschrieben. Dieses Verfahren stellt eine Abwandlung des bekannten Guareschi-Verfahrens-Umsetzung von Cyanacetamid mit Acetessigester – dar, bei dem jedoch die Herstellung des Cyanacetamids aus Chloressigsäure durch Veresterung, Ammonolyse des Esters zum Chloracetamid und Umsetzung dieses Produkts mit Natriumcyanid zum Cyanacetamid vorgelagert ist. Es ist auch bereits beschrieben worden [vgl. Journal of Organic Chemistry, Band 25 (1960), Seiten 560–564], Acetessigsäureäthylester und Cyanacetamid in Gegenwart von Piperidin oder Kaliumhydroxyd zum 3-Cyan-2,6-dihydroxy-4-methylpyridin zu kondensieren, um die Ausbeute des wertvollen Endprodukts zu erhöhen. Schliesslich ist aus der DE-OS 19 56 142 ein Verfahren zur Herstellung von N-alkylsubstituierten 6-Hydroxypyrid-2-onen bekannt, bei dem Cyanessigsäureäthylester, Acetessigsäureäthylester und ein Alkylamin in alkanolischer Lösung bei 110 bis 120 °C in einem Autoklav zur Reaktion gebracht werden. Das Amin wird hierbei in reiner Form eingesetzt, und nach Beendigung der Reaktion muss das als Lösungsmittel verwendete Alkanol abdestilliert werden. Wird nach den Angaben dieser Druckschrift beispielsweise das 1,4-Dimethyl-3-cyan-6-hydroxy-pyrid-2-on durch Umsetzung von 1 Mol Cyanessigsäureäthylester, 1 Mol Acetessigsäureäthylester, 2 Mol Methylamin und ca. 700 ml N-Butanol als Lösungsmittel bei einer Temperatur von 115 °C hergestellt, so erhält man nach einer Reaktionszeit von 4 Stunden etwa 70%, nach einer Reaktionszeit von 8 Stunden ca. 79% und nach einer Reaktionszeit von 16 Stunden ca. 80% Ausbeute. Es zeigt sich hierbei, dass dieses bekannte Verfahren offenbar einem Ausbeutegrenzwert bei etwa 80% der Theorie zustrebt, der auch bei erheblich verlängerter Reaktionszeit nicht überschritten wird. Abgesehen davon, dass Reaktionszeiten von 8 oder 16 Stunden zu einer sehr schlechten Raum-Zeit-Ausbeute des Verfahrens führen, sind auch 80% der Theorie für ein technisches Verfahren noch unbefriedigend, da nicht nur ein Verlust an Endprodukt in Kauf zu nehmen ist, sondern auch die 20% an Ausgangsmaterialien und Nebenprodukten auf ökologisch einwandfreie Weise zu beseitigen sind, was zusätzliche Kosten hervorruft.

Im Hinblick auf den grossen Wert der Verbindungen der allgemeinen Formel I war daher ein Herstellungsverfahren, das möglichst hohe Ausbeuten liefert, dringend erwünscht.

Überraschenderweise wurde nun gefunden, dass man 6-Hydroxypyrid-2-one der allgemeinen Formel I in wesentlich verbesserten Ausbeuten und in sehr guter Reinheit dann erhält, wenn man

Cyanacetamide der allgemeinen Formel II oder ein im wesentlichen Cyanacetamide der allgemeinen Formel II enthaltendes Substanzgemisch mit Acetessigsäureestern der allgemeinen Formel III in Gegenwart von mindestens einem Mol eines Amins der allgemeinen Formel IV in wässriger Lösung oder Suspension unter einem Druck von 0,5 bis 50 bar auf Temperaturen zwischen 50 und 200 °C erwärmt. Hierbei steht R in den allgemeinen Formeln I, II und IV für Wasserstoff oder einen gegebenenfalls verzweigten Alkylrest mit 1 bis 4 Kohlenstoffatomen. Solche für R stehende Alkylreste mit 1 bis 4 Kohlenstoffatomen sind Methyl, Äthyl, Propyl-1, Propyl-2, Butyl-1, Butyl-2, 2-Methylpropyl-(1) und 2-Methylpropyl-(2).

Ein wesentliches Charakteristikum des erfindungsgemässen Verfahrens ist die Anwesenheit von Wasser. Die verwendete Wassermenge beträgt mindestens 10 Gewichtsprozent bezogen auf das Gewicht des eingesetzten Cyanacetamids. Aus wirtschaftlichen Gründen werden in der Regel 10 bis 1000 Gewichtsprozent, vorzugsweise 50 bis 500 Gewichtsprozent, Wasser eingesetzt.

In den für das erfindungsgemässe Verfahren eingesetzten Cyanacetamiden der allgemeinen Formel II hat R jeweils dieselbe Bedeutung wie in dem herzustellenden 6-Hydroxypyrid-2-on.

Die als Ausgangsmaterialien verwendeten Cyanacetamide der allgemeinen Formel II sind bekannte Verbindungen und können auf einfache Weise nach literaturbekannten Verfahren hergestellt werden [z.B. B.B.Corson, R.. Scott U.C.E. Vose, Organic Syntheses, Coll. Vol. I, 179 (1941); H. Henecka U.P. Kurtz, Houben-Weyl, Methoden der Organischen Chemie, Band 8, Seite 658 (1952)]. Nach diesen bekannten Verfahren können die Cyanacetamide der allgemeinen Formel II dadurch erhalten werden, dass man Cyanessigsäureester mit Aminen der allgemeinen Formel $NH_2$-R unter Abspaltung von Alkoholen umsetzt.

In den Acetessigestern der allgemeinen Formel III, die für das erfindungsgemässe Verfahren als Ausgangsmaterial benutzt werden, bedeutet $R_1$ einen aliphatischen Rest mit 1 bis 8 Kohlenstoffatomen oder einen cycloaliphatischen Rest mit 3 bis 6 Kohlenstoffatomen. Diese Reste können im Prinzip gesättigt oder ungesättigt, verzweigt oder unverzweigt sein. Die Verwendung von Acetessigestern, in denen $R_1$ ungesättigt ist, kann jedoch zu Komplikationen führen, weil derartige ungesättigte Verbindungen zur Polymerisation neigen und daher Nebenreaktionen nicht auszuschliessen sind. Die Verwendung von im Alkoholrest ungesättigten Acetessigestern wird daher in der Regel vermieden. Normalerweise werden solche Acetessigester der allgemeinen Formel III eingesetzt, in denen $R_1$ einen Alkylrest mit 1 bis 8 Kohlenstoffatomen oder einen Cycloalkylrest mit 3 bis 6, vorzugsweise 5 oder 6, Kohlenstoffatomen bedeutet. Aus Gründen der technischen Zugänglichkeit werden vorzugsweise Acetessigester der allgemeinen Formel III eingesetzt, in denen $R_1$ einen niederen Alkylrest mit 1 bis 4 Kohlenstoffatomen, insbesondere einen Methyl- oder Äthylrest, bedeutet.

Bezogen auf 1 Mol Cynacetamid der allgemeinen Formel II werden bei der Durchführung des erfindungsgemässen Verfahrens 1,0 bis 1,5 Mol, vorzugsweise 1,0 bis 1,3 Mol, Acetessigsäureester der allgemeinen Formel III eingesetzt.

Gemäss der Bedeutung von R in dem herzustellenden 6-Hydroxypyrid-2-on werden Amine der allgemeinen Formel IV eingesetzt, in denen R Wasserstoff oder eine gegebenenfalls verzweigte Alkylgruppe mit 1 bis 4 Kohlenstoffatomen bedeutet. Solche Amine sind Monomethyl-, Monoäthyl-, Monopropyl-, Monoisopropyl Monobutyl-1-, Monobutyl-2-, Monoisobutyl- oder Mono-tert.-butylamin. Die genannten Amine können in reiner Form, oder, soweit sie wasserlöslich sind, auch in Form ihrer konzentrierten wässrigen Lösungen eingesetzt werden. Da bei der Herstellung von Ammoniak oder niederen Alkylaminen in der Regel zunächst wässrige Lösungen anfallen, ist die Verwendung solcher Lösungen für die Durchführung des erfindungsgemässen Verfahrens besonders vorteilhaft und bevorzugt.

Wird die erfindungsgemässe Reaktion unter Verwendung von wasserfreien Aminen der allgemeinen Formel IV durchgeführt, so ist die oben genannte erforderliche Wassermenge dem Reaktionsgemisch getrennt zuzusetzen. Bei der bevorzugten Verwendung von wässrigen Aminlösungen kann der getrennte Wasserzusatz je nach der Menge des mit der Aminlösung eingebrachten Wassers reduziert werden oder ganz unterbleiben. Die wässrigen Aminlösungen können 5 bis 80, vorzugsweise 20 bis 50, Gewichtsprozent des einzusetzenden Amins enthalten.

Pro Mol eingesetztes Cyanacetamid der allgemeinen Formel II muss mindestens 1 Mol des Amins der allgemeinen Formel IV eingesetzt werden. Die Menge des Amins ist nach oben hin nicht begrenzt. Aus wirtschaftlichen Gesichtspunkten werden jedoch 1,0 bis 2 Mol, vorzugsweise 1,0 bis 1,15 Mol, Amin verwendet.

Ein besonderer Vorteil des erfindungsgemässen Verfahrens besteht darin, dass die als Ausgangsmaterial einzusetzenden Cyanacetamide nicht unbedingt in reiner Form verwendet werden müssen, sondern ebensogut in Mischung mit anderen chemischen Substanzen, soweit diese gegen die Reaktanten des Verfahrens inert sind, vorliegen können. Solche Mischungen sollen im wesentlichen, d.h. mehr als 50 Gewichtsprozent, Cyanacetamide der allgemeinen Formel II enthalten. Insbesondere können solche im wesentlichen Cyanacetamide der allgemeinen Formel II enthaltende Gemische bei dem erfindungsgemässen Verfahren eingesetzt werden, die bei der Reaktion von Cyanessigestern mit Aminen der allgemeinen Formel IV als rohe Reaktionsmischungen erhalten werden. Diese Reaktionsmischungen enthalten neben dem erforderlichen Cyanacetamid der allgemeinen Formel II noch einen Überschuss von Aminen der allgemeinen Formel IV, die bei der Umsetzung des Cyanessigesters mit dem Amin entstandene aliphatische Hydroxyverbindung $R_1OH$ und eventuell geringe Mengen von Nebenprodukten.

Der Einsatz derartiger roher Umsetzungsgemische, die im wesentlichen Cyanacetamide der allgemeinen Formel II enthalten, anstelle der reinen Verbindungen bringt den Vorteil mit sich, dass der gesamte Aufwand zur Isolierung und Reinigung dieser Cyanacetamide vermieden werden kann, und man zudem von einem preisgünstigeren, weil einfacher herstellbaren Ausgangsmaterial, den Cyanessigsäureestern, ausgeht. Da der Einsatz dieser rohen Umsetzungsgemische zudem keine oder praktisch keine Verminderung der Ausbeute an 6-Hydroxy-pyrid-2-onen der allgemeinen Formel I mit sich bringt, ist die Durchführung des erfindungsgemässen Verfahrens unter Verwendung der im wesentlichen Cyanacetamide der allgemeinen Formel II enthaltenden rohen Reaktionsmischungen bevorzugt.

Zur Durchführung des erfindungsgemässen Verfahrens werden die Reaktionsteilnehmer in den oben angegebenen Mengenverhältnissen gemischt und die Mischung in einem druckfesten verschlossenen Gefäss auf eine Temperatur zwischen 50 und 200 °C, vorzugsweise zwischen 80 und 120 °C, erwärmt. Die Reaktion kann unter dem Druck eines inerten Fremdgases bei 0,5 bis 50 bar durchgeführt werden. Bevorzugt ist es, die Reaktion unter dem Eigendruck des Reaktionssystems bei der gewählten Reaktionstemperatur auszuführen. Dieser Eigendruck kann je nach den Reaktionspartnern und der gewählten Reaktionstemperatur zwischen 0,5 und 50 bar liegen. Im bevorzugten Temperaturbereich wird ein Druck von 1 bis 15 bar erreicht. Zur Aufrechterhaltung einer gleichmässigen Stoffverteilung in der Reaktionsmischung, insbesondere, wenn in Suspension gearbeitet wird, ist es zweckmässig, für eine dauernde Durchmischung des Ansatzes, z.B. durch Rühren, zu sorgen.

Will man das erfindungsgemässe Verfahren unter Einsatz rohrer, im wesentlichen Cyanacetamide der allgemeinen Formel II enthaltender Reaktionsmischungen ausführen, so wird zunächst ein Cyanessigsäureester der allgemeinen Formel V

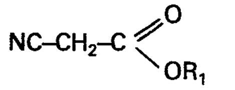 (V)

worin $R_1$ die vorgenannte Bedeutung hat, mit – bezogen auf 1 Mol des Esters – mindestens 1 Mol, vorzugsweise 1 bis 1,15 Mol, eines Amins der allgemeinen Formel IV, insbesondere in Form einer 5 bis 80 gewichtsprozentigen, vorzugsweise 20 bis 50 gewichtsprozentigen, wässrigen Lösung umgesetzt, wobei Temperaturen zwischen 0 und 100 °C, vorzugsweise 5 bis 80 °C angewendet werden.

Während der Reaktion wird vorteilhafterweise für eine gute Durchmischung, z.B. durch Rühren, Sorge getragen. Je nach der Reaktionsfähigkeit der eingesetzten Ester der allgemeinen Formel V und Amine der allgemeinen Formel IV sowie der gewählten Reaktionstemperatur ist die Reaktionsdauer bis zum praktisch vollständigen Umsatz 1 bis ca. 12 Stunden. So benötigt beispielsweise die Umsetzung von Cyanessigsäureäthylester mit einer 40%igen wässrigen Lösung von Methylamin bei 5 bis 10 °C eine Stunde, die entsprechende Umsetzung mit einer 50%igen Lösung von N-Butylamin bei 20 bis 30 °C 12 Stunden, bei 60 bis 80 °C 3 Stunden. Der Fortgang der Reaktion kann durch Dünnschicht-Chromatographie verfolgt werden.

Nach erfolgter Bildung des Cyanessigsäureamids wird dem Reaktionsgemisch nochmals mindestens 1 Mol, vorzugsweise 1 bis 1,15 Mol, des gleichen Amins bei einer Temperatur zwischen 0 und 30 °C zugefügt. Anschliessend setzt man dem Ansatz 1 bis 1,5 Mol, vorzugsweise 1 bis 1,3 Mol, eines Acetessigsäureesters der allgemeinen Formel III, vorzugsweise Acetessigsäuremethyl- bzw. -äthylesters, pro Mol eingesetzten Cyanessigesters ebenfalls bei Temperaturen zwischen 0 und 30 °C, gegebenenfalls unter Kühlung, zu.

Es ist auch möglich, die gesamte Aminmenge von mindestens 2 Mol, vorzugsweise 2 bis 2,3 Mol, von Anfang an vorzulegen, da dies nicht nur technisch einfacher, sondern auch bei der Umsetzung mit dem Cyanessigester kinetisch vorteilhafter ist. Wenn alle Reaktionsteilnehmer in der angegebenen Weise vereinigt worden sind, wird das Reaktionsgemisch, wie oben beschrieben, in einem druckfesten, verschlossenen Gefäss auf eine Temperatur zwischen 50 und 200 °C gebracht und so lange bei dieser Temperatur, zweckmässigerweise unter Rühren, gehalten, bis die Umsetzung zu dem gewünschten 6-Hydroxypyrid-2-on abgeschlossen ist.

Die Reaktionszeit liegt üblicherweise zwischen zwei und sechs Stunden.

Die Isolierung der 6-Hydroxypyrid-2-one erfolgt – gegebenenfalls nach Verdünnen der Reaktionslösung bzw. -suspension mit Wasser – durch Ansäuern mit Mineralsäuren, vorzugsweise Salzsäure oder Schwefelsäure, bei Temperaturen zwischen 0 und 100 °C, vorzugsweise 20 und 70 °C, gegebenenfalls Kühlen des Gemisches auf 0 bis 30 °C, und nachfolgendes Absaugen des ausgefallenen Produktes.

Will man das in Form des entsprechenden Ammoniumsalzes des jeweiligen 6-Hydroxypyrid-2-ons gebundene sowie das gegebenenfalls im Überschuss eingesetzte Amin zurückgewinnen, so setzt man nach beendeter Druckreaktion dem Reaktionsgemisch 2,0 bis 5,0 Mol Alkalihydroxyd zu, gegebenenfalls als wässrige Lösung, und destilliert anschliessend 1,0 bis 2,0 Mol Amin ab, zweckmässigerweise in eine mit Wasser beschickte Vorlage, sodass unmittelbar eine für weitere Ansätze verwendbare wässrige Aminlösung entsteht.

Aus dem wässrigen Destillationsrückstand kann dann, wie oben angegeben, das 6-Hydroxypyrid-2-on durch Ansäuren, gegebenenfalls Kühlen und Absaugen, isoliert werden.

Das erfindungsgemässe Verfahren führt gegenüber dem nächstvergleichbaren Verfahren der DE–OS 19 56 142 zu einer wesentlichen Ausbeutesteigerung, so z.B. im Fall der Herstellung des 1,4-Dimethyl-3-cyan-6-hydroxypyrid-2-ons zu einer

Ausbeutesteigerung von ca. 12 bis 15% der Theorie. Verbunden mit dieser Ausbeutesteigerung ist ein Rückgang der zu beseitigenden Nebenprodukte um weit über 50%. Das erfindungsgemässe Verfahren arbeitet mit kürzeren Reaktionszeiten und höheren Konzentrationen und gibt daher erheblich bessere Raum-Zeit-Ausbeuten als die bekannten Verfahren. Da das erfindungsgemässe Verfahren im wässrigen Medium ausgeführt wird, entfällt die Rückgewinnung organischer Lösungsmittel, wie z.B. die Rückgewinnung des n-Butanols durch Wasserdampfdestillation, wie es nach dem nächstvergleichbaren bekannten Verfahren erforderlich ist. Gleichzeitig mit der Einsparung der Wiedergewinnung von organischen Lösungsmitteln ergibt sich auch der Vorteil der grösseren Umweltfreundlichkeit, da bei dem erfindungsgemässen Verfahren auch keine sonst unvermeidlichen Lösungsmittelverluste entstehen können. Die Möglichkeit, wässrige Aminlösungen einzusetzen, ist ebenfalls technisch vorteilhaft, insbesondere deshalb, weil bei der Rückgewinnung der Aminüberschüsse wiederum wässrige Lösungen entstehen, die unmittelbar wieder in das Verfahren eingeschleust werden können und somit weder eine Umweltbelastung noch ein besonderer Aufwand für die ordnungsgemässe Abfallbeseitigung entsteht.

Im Hinblick auf den Stand der Technik war es höchst überraschend und nicht vorherzusehen, dass die Reaktionen des erfindungsgemässen Verfahrens in Gegenwart von Wasser glatt und fast vollständig ablaufen. Das Gegenteil wäre theoretisch zu erwarten gewesen, da die genannten Reaktionen in Gegenwart von Wasser durch das Auftreten von Nebenreaktionen beeinträchtigt werden könnten. Eine weitere überraschende Feststellung war es, dass das erfindungsgemässe Verfahren ohne Komplikationen auch unter Einsatz roher Cyanacetamidgemische durchgeführt werden kann.

Die nachfolgenden Ausführungsbeispiele veranschaulichen die Durchführung des erfindungsgemässen Verfahrens. Teile sind Gewichtsteile und Prozentangaben sind Gewichtsprozente.

Beispiel 1

In einem Autoklav werden 250 Teile Wasser, 98 Teile N-Methylcyanacetamid und 169 Teile Acetessigsäureäthylester vorgelegt, unter Rühren 40 Teile gasförmiges Monomethylamin zugedrückt und das Reaktionsgemisch danach 3 Stunden auf 90 °C erhitzt, wobei ein Druck von 2,9 bar entsteht. Man verdünnt den Ansatz mit 400 Teilen Wasser, setzt bei 30–35 °C 80 Teile 61%ige Schwefelsäure zu, kühlt auf 20 °C ab und saugt den ausgefallenen Niederschlag ab. Man erhält 155,8 Teile (95% der Theorie) 1,4-Dimethyl-3-cyano-6-hydroxypyrid-2-on vom Schmelzpunkt 285 °C.

Führt man die Reaktion unter einem Stickstoffüberdruck von 10 bzw. 20 bar durch, so gelangt man zu ähnlichen Ergebnissen.

Vergleichbare Resultate werden auch erhalten, wenn man anstelle von N-Methylcyanacetamid und Monomethylamin äquivalente Mengen an N-Äthyl- bzw. N-Isopropylcyanacetamid und Äthyl- bzw. Isopropylamin einsetzt.

Beispiel 2

Zu 162 Teilen einer 44,1%igen wässrigen Monomethylaminlösung fügt man unter Rühren bei 5–10 °C 99 Teile Cyanessigsäuremethylester hinzu, rührt eine Stunden nach, lässt 133,5 Teile Acetessigsäuremethylester zulaufen und erhitzt in einem Autoklav 4 Stunden auf 90 °C, wobei ein Druck von maximal 1,3 bar entsteht. Danach gibt man zur Reaktionslösung 296 Teile 27%ige Natronlauge und destilliert bei 85 °C 33 Teile Monomethylamin (46,2% der Theorie bezogen auf Einsatz) in eine mit ca. 42 Teilen Eiswasser beschickte Vorlage, so dass eine für erneute Umsetzungen geeignete, ca. 44%ige wässrige Monomethylaminlösung ensteht. Danach fällt man das 1,4-Dimethyl-3-cyano-6-hydroxypyrid-2-on durch Einfliessen in eine Mischung aus 100 Teilen Eis und 217 Teilen 61%ige Schwefelsäure aus. Das Produkt wird abgesaugt, mit Wasser gewaschen und getrocknet.

Ausbeute: 155,8 Teile (95% der Theorie) vom Schmelzpunkt 285 °C.

Setzt man anstelle des Cyanessigsäuremethylesters die äquivalente Menge Cyanessigsäurebutyl- bzw. -isooctylester ein, so gelangt man zu ähnlichen Ergebnissen.

Beispiel 3

Zur Herstellung des 3-Cyano-4-methyl-6-hydroxypyrid-2-ons lässt man zu 160 Teilen 24%igem wässrigen Ammoniak unter Rühren bei 10 bis 15 °C 99 Teile Cyanessigsäuremethylester zulaufen. Man rührt ca. 1 Stunde bei 20 °C nach, setzt anschliessend 133,5 Teile Acetessigsäuremethylester zu und erhitzt das Reaktionsgemisch in einem Autoklav 4 Stunden auf 80 °C, wobei ein Druck von maximal 1,5 bar entsteht. Danach verdünnt man die Reaktionssuspension mit 400 Teilen Wasser und setzt bei 50–60 °C 114 Teile 61%ige Schwefelsäure hinzu und kühlt auf 20 °C ab. Das ausgefallene Produkt wird abgesaugt, mit Wasser gewaschen und getrocknet. Man erhält so 147,0 Teile (98% der Theorie) 3-Cyano-4-methyl-6-hydroxypyrid-2-on vom Schmelzpunkt über 305 °C.

Verwendet man anstelle des 24%igen wässrigen Ammoniaks eine 5-bzw. 15- oder 20%ige wässrige Ammoniaklösung, so gelangt man zu ähnlichen Ergebnissen.

Beispiel 4

Zu 184,4 Teilen einer 49,4%igen wässrigen Äthylaminlösung setzt man unter Rühren bei 5–15 °C 99 Teile Cyanessigsäuremethylester zu, rührt eine Stunde nach, fügt 133,5 Teile Acetessigsäuremethylester hinzu und erhitzt in einem Autoklav 5 Stunden auf 100 °C, wobei ein Druck von 10,5 bar entsteht. Danach verdünnt man die Reaktionslösung mit 600 Teilen Wasser und säuert mit 74 Teilen 61%iger Schwefelsäure an. Das ausgefallene Produkt wird abgesaugt, gewaschen und getrocknet. Man erhält so 153 Teile (85,9% der

Theorie) 1-Äthyl-3-cyano-4-methyl-6-hydroxypyrid-2-on vom Schmelzpunkt 242–45 °C.

Verwendet man anstelle des Acetessigsäuremethylesters die äquivalente Menge an Acetessigsäurepropyl- bzw. -cyclohexylester, so werden ähnliche Resultate erhalten.

Beispiel 5

117,5 Teile n-Butylamin werden unter Rühren bei 20 bis 30 °C mit 69,3 Teilen Cyanessigsäuremethylester versetzt, ca. 12 Stunden verrührt, 150 Teile Wasser und 93,4 Teile Acetessigsäuremethylester hinzugefügt und in einem Autoklav 6 Stunden auf 120 °C erhitzt, wobei ein Druck von 13 bar entsteht. Anschliessend lässt man die Reaktionslösung in ein Gemisch aus 400 Teilen Wasser und 75 Teilen 61%iger Schwefelsäure einlaufen, saugt den ausgefallenen Niederschlag ab, wäscht mit Wasser nach und trocknet. Man erhält 123 Teile (85% der Theorie) 1-Butyl-3-cyano-4-methyl-6-hydroxypyrid-2-on vom Schmelzpunkt 220 °C.

Setzt man analog obigem Beispiel statt n-Butylamin n-Propyl- bzw. Isopropylamin ein – gegebenenfalls in Form einer 5–80%igen wässrigen Lösung – so isoliert man das entsprechende 1-Propyl- bzw. 1-Isopropyl-3-cyano-6-hydroxypyrid-2-on vom Schmelzpunkt 239–240 °C bzw. 252 °C.

**Patentansprüche**

1. Verfahren zur Herstellung von 6-Hydroxypyrid-2-onen der allgemeinen Formel I

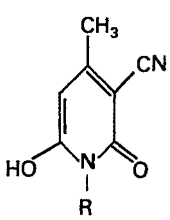

(I)

worin R für Wasserstoff oder einen gegebenenfalls verzweigten Alkylrest mit 1 bis 4 Kohlenstoffatomen steht, durch Umsetzung eines Cyanacetamids der allgemeinen Formel II

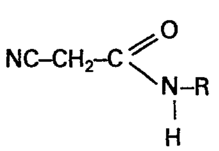

(II)

mit einem Acetessigsäureester der allgemeinen Formel III

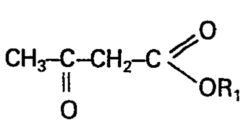

(III)

worin $R_1$ einen aliphatischen Rest mit 1 bis 8 Kohlenstoffatomen oder einen cycloaliphatischen Rest mit 3 bis 6 Kohlenstoffatomen bezeichnet, dadurch gekennzeichnet, dass man die Umsetzung in Gegenwart von mindestens einem Mol eines Amins der allgemeinen Formel IV

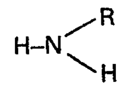

(IV)

worin R die vorgenannte Bedeutung hat, in wässriger Lösung oder Suspension unter einem Überdruck von 0,5 bis 50 bar bei Temperaturen zwischen 50 und 200 °C durchführt.

2. Verfahren gemäss Anspruch 1, dadurch gekennzeichnet, dass man ein im wesentlichen ein Cyanacetamid der allgemeinen Formel II enthaltendes Substanzgemisch einsetzt, das erhalten wurde durch Umsetzung eines Cyanessigsäureesters der allgemeinen Formel V

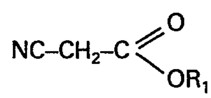

(V)

worin $R_1$ die vorgenannte Bedeutung hat, mit einem Amin der allgemeinen Formel IV.

3. Verfahren gemäss den Ansprüchen 1 oder 2, dadurch gekennzeichnet, dass der Arbeitsdruck bei der Reaktion der Reaktanten der allgemeinen Formeln II, III und IV der bei der Arbeitstemperatur entstehende Gesamtdruck des Reaktionssystems ist.

4. Verfahren gemäss den Ansprüchen 1 bis 3, dadurch gekennzeichnet, dass die Arbeitstemperatur bei der Reaktion der Reaktanten der allgemeinen Formeln II, III und IV zwischen 80 und 120 °C liegt.

5. Verfahren gemäss den Ansprüchen 1 bis 4, dadurch gekennzeichnet, dass R Wasserstoff, Methyl oder Äthyl bedeutet.

6. Verfahren nach den Ansprüchen 1 bis 5, dadurch gekennzeichnet, dass $R_1$ Alkyl mit 1 bis 8 C-Atomen oder Cycloalkyl mit 3 bis 6 C-Atomen bedeutet.

7. Verfahren gemäss den Ansprüchen 1 bis 6, dadurch gekennzeichnet, dass $R_1$ Alkyl mit 1 bis 4 C-Atomen bedeutet.

**Revendications**

1. Procédé de préparation d'hydroxy-6 pyridones-2 répondant à la formule générale I

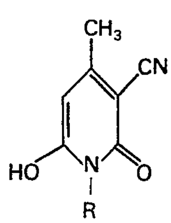

(I)

dans laquelle R représente l'hydrogène ou un radical alkyle, éventuellement ramifié, contenant de 1 à 4 atomes de carbone, par réaction d'un cyanacétamide répondant à la formule générale II

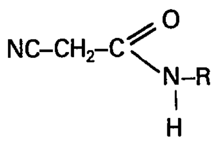 (II)

avec un ester de l'acide acétylacétique répondant à la formule générale III

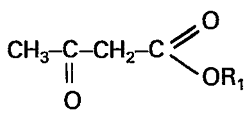 (III)

dans laquelle $R_1$ représente un radical aliphatique contenant de 1 à 8 atomes de carbone ou un radical cycloaliphatique contenant de 3 à 6 atomes de carbone, procédé caractérisé en ce qu'on effectue la réaction en présence d'au moins une mole d'une amine répondant à la formule générale IV

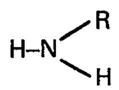 (IV)

dans laquelle R a la signification précédemment donnée, en suspension ou solution aqueuse, sous une surpression de 0,5 à 50 bar et à des températures comprises entre 50 et 200 °C.

2. Procédé selon la revendication 1 caractérisé en ce qu'on met en jeu un mélange contenant essentiellement un cyanacétamide de formule générale II et qui a été obtenu par réaction d'un ester cyanacétique répondant à la formule générale V

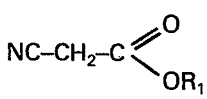 (V)

dans laquelle $R_1$ a la signification précédemment donnée, avec une amine de formule générale IV.

3. Procédé selon l'une des revendications 1 et 2 caractérisé en ce que la pression à laquelle on opère lors de la réaction des composés de formules générales II, III et IV est la pression totale du système réactionnel qui s'établit à la température opératoire.

4. Procédé selon l'une quelconque des revendications 1 à 3 caractérisé en ce que la température à laquelle on opère lors de la réaction des composés de formules générales II, III et IV est comprise entre 80 et 120 °C.

5. Procédé selon l'une quelconque des revendications 1 à 4 caractérisé en ce que R représente l'hydrogène, un radical méthyle ou un radical éthyle.

6. Procédé selon l'une quelconque des revendications 1 à 5 caractérisé en ce que $R_1$ représente un radical alkyle contenant de 1 à 8 atomes de carbon ou un radicalt cycloalkyle contenant de 3 à 6 atomes de carbone.

7. Procédé selon l'une quelconque des revendications 1 à 6 caractérisé en ce que $R_1$ représente un radicalt alkyle contenant de 1 à 4 atomes de carbone.

**Claims**

1. Process for the preparation of 6-hydroxypyrid-2-ones of the general formula

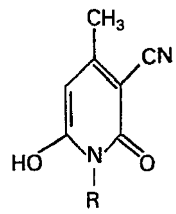 (I)

wherein R denotes hydrogen or an optionally branched alkyl moiety having 1 to 4 carbon atoms, by reacting a cyanoacetamide of the general formula II

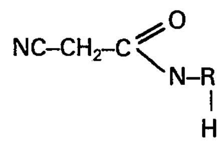 (II)

with an acetoacetic acid ester of the general formula III

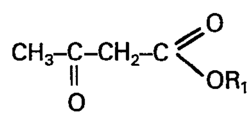 (III)

wherein R denotes an aliphatic moiety having 1 to 8 carbon atoms or a cycloaliphatic moiety having 3 to 6 carbon atoms, characterised in that the reaction is carried out in the presence of at least one mole of an amine of the general formula IV

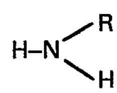 (IV)

wherein R has the meaning indicated above, in an aqueous solution or suspension at an excess pressure of 0,5 to 50 bars and at temperatures from 50 to 200 °C.

2. Process according to claim 1 characterised in that a mixture is employed which essentially contains a cyanoacetamide of the general formula II and which was obtained by reacting a cyanoacetic acid ester of the general formula V

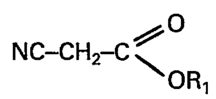 (V)

wherein $R_1$ has the meaning indicated above, with an amine of the general formula IV.

3. Process according to claim 1 or 2, characterised in that in the reaction of the reactants of the general formulae II, III and IV the operating pressure is the total pressure of the reaction system generated at the operating temperature.

4. Process according to claims 1 to 3, characterised in that in the reaction of the reactants of the

general formulae II, III and IV the operating temperature is between 80 and 120 °C.

5. Process according to claims 1 to 4, characterised in that R denotes hydrogen, methyl or ethyl.

6. Process according to claims 1 to 5, characterised in that $R_1$ denotes alkyl having 1 to 8 carbon atoms or cycloalkyl having 3 to 6 carbon atoms.

7. Process according to claims 1 to 6, characterised in that $R_1$ denotes alkyl having 1 to 4 carbon atoms.